(19) Europäisches Patentamt
European Patent Office
Office européen des brevets

(11) Numéro de publication: **0 049 555**
**B1**

(12) # FASCICULE DE BREVET EUROPEEN

(45) Date de publication du fascicule du brevet: **22.02.84**

(21) Numéro de dépôt: **81201342.3**

(22) Date de dépôt: **14.11.79**

(60) Numéro de publication de la demande initiale en application de l'article 76 CBE: **0012639**

(51) Int. Cl.³: **C 07 D 215/56**
//C07D417/12, C07D401/12, C07D413/12, C07C101/28, C07C119/18

(54) **Nouveaux dérivés de l'acide 2-trifluorométhyl-4-hydroxy-3-quinoline carboxylique et leur procédé de préparation.**

(30) Priorité: **08.12.78 FR 7834592**

(43) Date de publication de la demande:
**14.04.82 Bulletin 82/15**

(45) Mention de la délivrance du brevet:
**22.02.84 Bulletin 84/8**

(84) Etats contractants désignés:
**BE CH DE FR GB IT NL SE**

(56) Documents cités:
**FR - A - 2 324 304**
**FR - A - 2 340 735**

**JOURNAL OF MEDICINAL CHEMISTRY, volume 22, no. 7, juillet 1979 MINNESOTA UNIV. (US) ERICKSON H.E. et al. "Inhibition of rat passive cutaneous anaphylaxis by 3-(tetrazol-5-yl) quinolines", pages 816-23**

(73) Titulaire: **ROUSSEL-UCLAF**
**35, boulevard des Invalides**
**F-75007 Paris (FR)**

(72) Inventeur: **Clemence, François**
**2, rue Turgot**
**F-75009 Paris (FR)**
Inventeur: **Deraedt, Roger**
**23, Allée Jean-Baptiste Clément**
**F-93320 Pavillons Sous Bois (FR)**
Inventeur: **Allais, André**
**1, Allée Eugénie**
**F-93220 Gagny (FR)**
Inventeur: **Le Martret, Odile**
**42, Avenue de Versailles**
**F-75016 Paris (FR)**

(74) Mandataire: **Vieillefosse, Jean-Claude et al,**
**ROUSSEL-UCLAF Boîte postale no. 9 111, route de Noisy**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de l'acide 2-trifluorométhyl-4-hydroxy-3-quinoline carboxylique et leur procédé de préparation

La présente invention est une demande divisionnaire de la demande européenne publiée sous le n° 0012639. La présente invention concerne de nouveaux dérivés de l'acide 3-quinoléïne carboxylique et leur procédé de préparation. Ces produits correspondent aux produits de formule (II) et (VII) de la demande européene publiée sous le n° 0012639 qui sont les produits permettant de préparer les produits de formule (I') thérapeutiquement actifs, décrits dans ladite demande européenne.

Le brevet français n° 2340735 décrit des produits, appelés aussi produits de formule (II), dérivés de l'acide 3-quinoléïne carboxylique et intermédiaires de produits de formule (I) thérapeutiquement actifs.

Les produits de la présente demande sont différents des produits de formule (II) de brevet français n° 2340735 puisqu'ils sont substitués en 2 par un radical trifluorométhyle alors que les produits du brevet français n° 2340734 sont éventuellement substitués en 2 par un radical hydroxyle ou un radical alcoyle.

L'invention a pour objet les composés de formule (A):

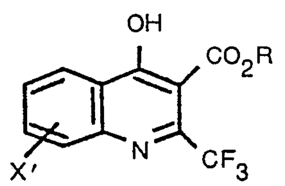

(A)

dans laquelle X' en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, et R représente un atome d'hydrogène, ou un radical alcoyle renfermant de 1 à 8 atomes de carbone.

Lorsque X' représente un atome d'halogène, il s'agit de préférence d'un atome de chlore.

Lorsque X' représente un radical alcoyle, il s'agit de préférence du radical méthyle, éthyle, n-propyle, n-butyle, n-pentyl, isopropyle ou isobutyle.

Lorsque X' représente un radical alcoxy, il s'agit de préférence du radical méthoxy, éthoxy ou n-propoxy.

Lorsque R représente un radical alcoyle, il s'agit de préférence du radical méthyle, éthyle, n-propyle.

L'invention a notamment pour objet les composés de formule (A) dans laquelle X' représente un radical trifluorométhyle et ceux pour lesquels X' est en position 8.

L'invention concerne également un procédé de préparation des composés de formule (A) caractérisé en ce que l'on soumet un composé de formule (II):

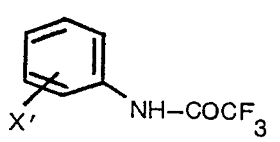

(II)

dans laquelle X' conserve la même signification que celle indiquée précédemment à l'action d'un agent de chloruration, obtient ainsi un composé de formule (III):

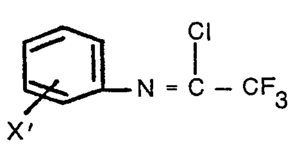

(III)

que l'on fait réagir avec un dérivé de formule:

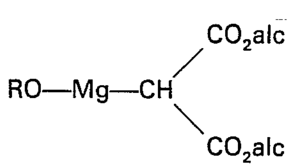

dans laquelle R et alc, identiques ou différents, représentent un radical alcoyle renfermant de 1 à 8 atomes de carbone, ou avec un malonate d'alcoyle de formule:

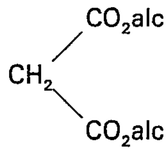

dans laquelle alc représente un radical alcoyle renfermant de 1 à 8 atomes de carbone, en présence d'une base forte, pour obtenir un composé de formule (IV):

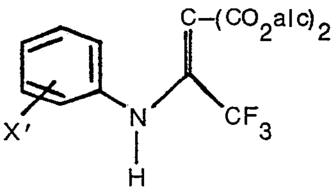

(IV)

que l'on cyclise pour obtenir un composé de formule (A) dans laquelle R représente un radical alcoyle renfermant de 1 à 8 atomes de carbone, que l'on saponifie le cas échéant pour obtenir un composé de formule (A) dans laquelle R représente un atome d'hydrogène.

Dans un mode de réalisation préférée du procédé de préparation des composés de formule (A):

—la réaction du composé de formule (II) avec l'agent de chloruration qui est, de préférence, le pentachlorure de phosphore ou le mélange triphénylphosphine—tétrachlorure de carbone, a lieu à une température comprise entre 60 et 120°C.

—Le dérivé de formule

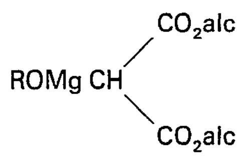

utilisé est un dérivé pour lequel R et alc représentent un radical méthyle, éthyle ou n-propyle.

—Le malonate d'alcoyle utilisé est le malonate d'éthyle, et la réaction avec le composé de formule (III) est effectuée en présence d'hydrure de sodium.

—La cyclisation du composé de formule (IV) est réalisée par chauffage, par exemple à une température comprisé entre 150°C et 250°C.

—On saponifie le composé de formule (A) dans laquelle R représente un radical alcoyle au moyen de la soude ou de la potasse.

Les composés de formule (II) utilisés comme produits de départ sont des produits connus d'une façon générale qui peuvent être préparés selon le procédé décrit dans *CA* 544430i.

Les composés deformule (A) de la présente demande sont soit des acides, soit des esters. Ils sont utilisés pour obtenir les composés de formule (I') thérapeutiquement actifs décrits dans la demande européenne publiée sous le n° 0012639, selon le procédé décrit dans cette demande. Dans ce procédé, le produit de formule (A) peut être aussi un dérivé fonctionnel d'un acide, comme par exemple, un chlorure d'acide, un anhydride ou un anhydride mixte.

Les exemples suivants illustrent l'invention sans toutefois la limiter.

Exemple 1

Préparation de l'acide 2,8-bis-(trifluorométhyl) 4-hydroxy 3-quinoléïne carboxylique et de son chlorure.

Stade A: chlorure de 2,2,2-trifluoro N-/2(trifluorométhyl) phényl/ éthanimidoyle.

On porte sous agitation à 80°C pendant 4 heures, 27,48 g de pentachlorure de phosphore et 30,84 g de 2,2,2,-trifluoro N-/N-(trifluorométhyl) phényl/ acétamide. On laisse refroidir. On purifie le produit ainsi obtenu par distillation, sous pression réduite. On obtient ainsi 15,2 g du produit recherché. Température d'ébullition 85°C sous 30 mm de mercure.

$n_D^{21}=1,4297$.

Stade B: 2-/2,2,2-trifluoro 1-/(2-trifluorométhyl) phényl amine/ éthylidène/ propane dioate d'éthyle.

On introduit sous agitation et sous courant d'azote 4,8 g d'hydrure de sodium à 61% dans 300 $cm_3$ de toluène, puis ajoute goutte à goutte à température ambiante 20,8 g de malonate d'ethyle en solution dans 40 $cm_3$ de toluène. On porte le mélange réactionnel à 90°C pendant 45 minutes. On

refroidit à 20°C, et ajoute 25 g du produit préparé au stade A dans 30 $cm_3$ de toluène. On porte au reflux pendant 4 heures. On refroidit, verse dans une solution glacée d'acide chlorhydrique, extrait à l'éther, lave à l'eau, sèche, filtre et concentre à sec. On obtient 45 g d'un produit que l'on purifie par chromatographie. On obtient ainsi 28,7 g du produit recherché.
$n_D^{21}$=1,4695.

Stade C: 2,8-bis (trifluorométhyl) 4-hydroxy 3-quinoléïne carboxylate d'éthyle.

On porte à 210°C pendant une heure environ 10 g du produit préparé au stade B, dans un ballon muni d'une tête de distillation. On laisse refroidir et obtient 8,75 g du produit recherché fondant à 68°C que l'on utilise tel quel dans le stade suivant.

Stade D: acide 2,8-bis-(trifluorométhyl) 4-hydroxy 3-quinoléïne carboxylique.

On porte au reflux pendant 7 heures 61,1 g du produit préparé au stade C dans une solution de 900 $cm_3$ d'éthanol et 300 $cm_3$ de soude 36°B. On chasse l'éthanol sous pression réduite, verse dans l'eau, extrait à l'éther et lave à l'eau. On acidifie la phase aqueuse avec de l'acide chlorhydrique. On glace, essore, lave et sèche les cristaux. On obtient 53,4 g du produit recherché fondant à 188°C.

Stade E: chlorure de l'acide 2,8-bis (trifluorométhyl) 4-hydroxy 3-quinoléïne carboxylique.

On ajoute sous agitation 9,6 $cm_3$ de chlorure de thionyle dans une suspension de 8,75 g d'acide 2,8-bis (trifluorométhyl)4-hydroxy 3-quinoléïne carboxylique dans 285 $cm_3$ de benzène. On porte le mélange réactionnel au reflux pendant une heure et demie. On évapore le benzène et l'excès de chlorure de thionyle. On reprend deux fois le résidu avec du benzène anhydre. On obtient ainsi le produit recherché.

## Exemple 2

Préparation de l'acide 2,7-bis-(trifluorométhyl) 4-hydroxy 3-quinoléïne carboxylique et de son chlorure.

Stade A: 2,2,2-trifluoro N-/3-(trifluorométhyl) phényl/acétamide.

A 124,97 g d'anhydride trifluoroacétique à 40°C, on ajoute, en 25 minutes, 80 g de métatrifluorométhylaniline et agite, pendant 2 heures, sous atmosphère inerte, à température ambiante. On concentre à sec sous pression réduite, reprend par du chlorure de méthylène, lave à l'eau avec une solution aqueuse de carbonate de potasse et amène à sec. On obtient 121,8 g de produit. On en recristallise 1,5 g dans l'éther de pétrole (éb. 60—80°C) pour obtenir 1,38 g d'amide fondant à 69°C.

Stade B: chlorure de 2,2,2-trifluoro N-/3-(trifluorométhyl) phényl/ éthanimidoyle.

On mélange intimement 120,3 g du produit obtenu précédemment avec 106,6 g de pentachlorure de phosphore à température ambiante sous atmosphère inerte, chauffe à 80°C pendant 5 heures, élimine l'oxychlorure de phosphore par distillation sous pression réduite (25°C/40 mm) et recueille 31,44 g de produit attendu (éb. 75°C/30 mm).

Stade C: 2-/2,2,2-trifluoro 1-/3-(trifluorométhyl) phénylamino/éthylidène/propane dioate d'éthyle.

On lave 0,86 g d'hydrure de sodium en dispersion à 50% dans l'huile, avec de l'éther de pétrole (éb. 60°—80°C) et le met en suspension dans 3,2 $cm_3$ de diméthylformamide sec. On ajoute à 17°C, en 30 minutes, sous atmosphère inerte, 3,25 g de malonate d'éthyle dans 3,3 $cm_3$ de diméthylformamide, agite, 1 heure à 20°C, ajoute en 30 minutes à 18°—20°C, 4 g du produit obtenu au stade précédent en maintenant la température inférieure à 23°C. On agite encore 1 heure à température ambiante, verse le mélange réactionnel sur 100 $cm_3$ d'eau glacée et extrait à l'éther éthylique, lave avec une solution aqueuse saturée de chlorure de sodium, sèche et concentre à sec. Après chromatographie sur silice du résidu, on élue au chlorure de méthylène et obtient 3,00 g de produit purifié.

Analyse: $C_{16}H_{15}O_4NF_6$=399,291

| | C% | H% | N% | F% |
|---|---|---|---|---|
| Calculé: | 48,13 | 3,78 | 3,50 | 28,54 |
| Trouvé: | 48,3 | 3,7 | 3,4 | 28,2 |

Stade D: 2,7-bis-(trifluorométhyl) 4-hydroxy 3-quinoléïne carboxylate d'éthyle.

On chauffe à 210°C, 32,62 g du produit obtenu, pendant 1 heure 30 minutes, sous pression atmosphérique, puis, 15 minutes sous pression réduite (30 mm). On refroidit, ajoute 10 $cm_3$ d'éther de pétrole, glace, essore et isole 24,5 g produit brut (F=123°C) qui, recristallisé dans l'isopropanol fond à 156°C.

Stade E: acide 2,7-bis-(trifluorométhyl) 4-hydroxy 3-quinoléïne carboxylique.

On mélange 21,38 g de produit obtenu au stade précédent dans 300 $cm_3$ d'éthanol et 60,5 $cm_3$ de lessive de soude, porte au reflux sous agitation pendant 7 heures 15 minutes et distille l'éthanol à 40°C sous pression réduite. On reprend le résidu par environ 300 $cm_3$ d'eau, amène à pH 1 avec de l'acide chlorhydrique à 20%, essore, lave à l'eau et reprend dans 350 $cm_3$ d'éther éthylique. Les phases éthérées sont lavées à l'eau, séchées, évaporées à sec sous pression réduite. On obtient 18,65 g de produit attendu (F=240°C).

Stade F: chlorure d'acide 2,7-bis-(trifluorométhyl) 4-hydroxy 3-quinoléïne carboxylique.

Dans 535 $cm_3$ de benzène anhydre, on met en suspension 17,246 g d'acide 2,7-bis-(trifluorométhyl) 4-hydroxy 3-quinoléïne carboxylique, ajoute 31,89 g de chlorure de thionyle et chauffe au reflux pendant 1 heure 45 minutes. On concentre à sec 40°C sous pression réduite, reprend par du benzène, évapore dans les mêmes conditions et obtient 18,62 g de produit attendu.

Exemple 3

Préparation de l'acide 2-(trifluorométhyl) 4-hydroxy 3-quinoléïne carboxylique et de son chlorure.

Stade A: 2,2,2-trifluoro N-phényl acétamide.

En opérant comme au stade A de l'exemple 2, à partir d'aniline, on obtient le produit attendu (F=85°C) qui, recristallisé dans l'éther de pétrole (éb. 60—80°C), fond à 87°C.

Stade B: chlorure de 2,2,2-trifluoro N-phényl éthanimidoyle.

On dissout 72,7 g du produit obtenu ci-dessus dans 770 $cm_3$ de chlorure de méthylène, ajoute à 20°C et sous atmosphère inerte, 118 g de triphénylphosphine et 44 $cm_3$ de tétrachlorure de carbone. Après 6 heures de reflux, on chasse le chlorure de méthylène par distillation sous pression atmosphérique, chromatographie sur silice le résidu, élue au chlorure de méthylène et isole 84,0 g de produit attendu. On purifie ce dernier par distillation et obtient 56,1 g de produit (éb.=70°C/20 mm).

Stade C: (1-phénylamino 2,2,2-trifluoro éthylidène) propanedioate d'éthyle.

A 12,8 g d'hydrure de sodium dans 140 $cm_3$ de diméthylformamide, on ajoute en 20 minutes à 20°C, 52,8 g de malonate d'éthyle dans 40 $cm_3$ de diméthylformamide. On agite pendant 30 minutes, refroidit à 5°C et ajoute 56 g du produit obtenu ci-dessus, en 45 minutes. On maintient l'agitation pendant 3 heures 30 minutes en laissant la température remonter à 20°C. On verse le mélange réactionnel sur 400 $cm_3$ d'acide chlorhydrique N dans la glace, extrait à l'éther éthylique, lave à l'eau jusqu'à neutralité, sèche, concentre à sec et obtient 98,0 g de produit attendu.

Stade D: 2-trifluorométhyl 4-hydroxy 3-quinoléïne carboxylate d'éthyle.

On opère comme indiqué au stade D de l'exemple 2 au départe de 96,0 g de produit obtenu ci-dessus et obtient 40,2 g de produit attendu (F=192°C).

Stade E: acide 2-trifluorométhyl 4-hydroxy 3-quinoléïne carboxylique.

On opère comme indiqué au stade E de l'exemple 2 à partir de 30 g de produit obtenu précédemment et sans purification dans l'éther, obtient 26,3 g de produit attendu. (F=262°C).

Stade F: chlorure d'acide 2-trifluorométhyl 4-hydroxy 3-quinoléïne carboxylique.

On opère comme indiqué au stade F de l'exemple 2 à partir de 12,85 g de produit obtenu au stade E précédent avec chauffage au reflux pendant 2 heures 30 minutes et obtient le produit attendu.

Exemple 4

Préparation de l'acide 2-trifluorométhyl 6-isopropyl 4-hydroxy 3-quinoléïne carboxylique et de son chlorure.

Stade A: 2,2,2-trifluoro N-(4-isopropyl phényl) acétamide.

On opère comme indiqué au stade A de l'exemple 2 au départ de 65 g de para isopropylaniline et de 121 g d'anhydride trifluoroacétique. On obtient 109 g de produit attendu. (F=98°C).

Stade B: chlorure de 2,2,2-trifluoro N-(4-isopropyl phényl) éthanimidoyle.

On opère comme indiqué au stade B de l'exemple 3 au départ de 96,9 g du produit obtenu ci-dessus et obtient 74,55 g de produit attendu. (éb. 110°C/20 mm).

Stade C: 2-/2,2,2-trifluoro 1-(4-isopropyl phénylamino) éthylidène/ propane dioate d'éthyle.

On opère comme indiqué au stade C de l'exemple 3 au départ de 74,5 g du produit obtenu ci-dessus et obtient 111,3 g de produit attendu brut, utilisé te quel pour la suite de la synthèse.

Stade D: 2-trifluorométhyl 6-isopropyl 4-hydroxy 3-quinoléïne carboxylate d'éthyle.

On cyclise 123,4 g de produit brut obtenu de la manière indiquée au stade C ci-dessus, en opérant selon le procédé décrit au stade D de l'exemple 3 et obtient, après lavage à l'hexane, 52,15 g de produit attendu. (F=95°C).

Stade E: acide 2-trifluorométhyl 6-isopropyl 4-hydroxy 3-quinoléïne carboxylique.

On opère comme indiqué au stade E de l'exemple 3 au départ de 25 g d'ester obtenu au stade D ci-dessus et obtient 22 g d'acide attendu. (F=206°C).

Stade F: chlorure d'acide 2-trifluorométhyl 6-isopropyl 4-hydroxy 3-quinoléïne carboxylique.

On opère comme indiqué au stade F de l'exemple 3 au départ de 20 g d'acide obtenu au stade E ci-dessus, et obtient le chlorure d'acide attendu.

Exemple 5

Préparation de l'acide 2-trifluorométhyl 6-méthoxy 4-hydroxy 3-quinoléïne carboxylique et de son chlorure.

Stade A: 2,2,2-trifluoro N-(3-méthoxyphényl) acétamide.

On opère comme indiqué au stade A de l'exemple 2 au départ de 65 g de para méthoxyamiline et de 120 g d'anhydride trifluoroacétique. On obtient 113 g de produit attendu. (F=115°C).

Stade B: chlorure de 2,2,2-trifluoro N-(3-méthoxy phényl) éthanimidoyle.

On opère comme indiqué au stade B de l'exemple 3 au départ de 62 g du produit obtenu au stade A ci-dessus et obtient 40 g de produit attendu (éb. 108°C/20 mm).

Stade C: 2-/2,2,2-trifluoro 1-(3-méthoxyphénylamino) éthylidène/ propane dioate d'éthyle.

On opère comme indiqué au stade C de l'exemple 3 au départ de 6 g de produit obtenu au stade B ci-dessus et obtient 5 g de produit attendu brut utilisé tel quel pour la suite de la synthèse.

Stade D: 2-trifluorométhyl 6-méthoxy 4-hydroxy 3-quinoléïne carboxylate d'éthyle.

On opère comme indiqué au stade D de l'exemple 3 au départ de 4,5 g du produit obtenu au stade C ci-dessus, et obtient 3,35 g de produit attendu. (F=184°C).

Stade E: acide 2-trifluorométhyl 6-méthoxy 4-hydroxy 3-quinoléïne carboxylique.

On opère comme indiqué au stade E de l'exemple 3 au départ de 2,85 g d'ester obtenu au stade D ci-dessus, et obtient 2,50 g d'acide attendu. (F=256°C).

Stade F: chlorure d'acide 2-trifluorométhyl 6-méthoxy 4-hydroxy 3-quinoléïne carboxylique.

On opère comme indiqué au stade F de l'exemple 3 au départ de 2 g d'acide obtenu au stade E ci-dessus et obtient le chlorure d'acide attendu.

**Revendications**

1. Les composés de formule (A):

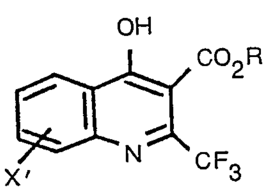

(A)

dans laquelle X' en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, et R représente un atome d'hydrogène, ou un radical alcoyle renfermant de 1 à 8 atomes de carbone.

2. Les composés de formule (A) tels que définis à la revendication 1, dans laquelle X' représente un radical trifluorométhyle.

3. Les composés de formule (A) tels que définis à la revendication 1 ou 2 pour lesquels X' est en position 8.

4. Procédé de préparation des composés de formule (A) caractérisé en ce que l'on soumet un composé de formule (II):

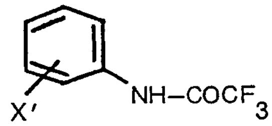

(II)

dans laquelle X′ conserve la même signification que celle definie à la revendication 1 à l'action d'un agent de chloruration, obtient ainsi un composé de formule (III):

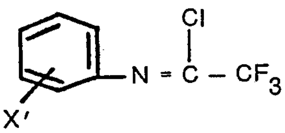

(III)

que l'on fait réagir avec un dérivé de formule:

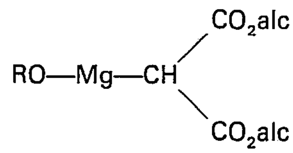

dans laquelle R et alc, identiques ou différents, représentent un radical alcoyle renfermant de 1 à 8 atomes de carbone, ou avec un malonate d'alcoyle de formule:

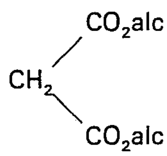

dans laquelle alc représente un radical alcoyle renfermant de 1 à 8 atomes de carbone, en présence d'une base forte, pour obtenir un composé de formule (IV):

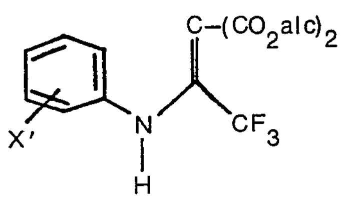

(IV)

que l'on cyclise pour obtenir un composé de formule (A) dans laquelle R représente un radical alcoyle renfermant de 1 à 8 atomes de carbone, que l'on saponifie le cas échéant pour obtenir un composé de formule (A) dans laquelle R représente un atome d'hydrogène.

**Claims**

1. Compounds with the formula (A):

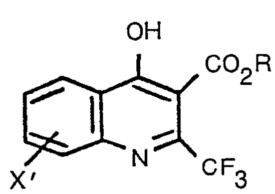

(A)

in which X′ in position 5, 6, 7 or 8 represents a hydrogen atom, a halogen atom, an alkyl radical linear or branched containing 1—5 carbon atoms, an alkoxy radical linear or branched containing 1—4 carbon atoms, a trifluoromethyl radical, a trifluoromethylthio radical or a trifluoromethoxy radical, and R represents a hydrogen atom or an alkyl radical with 1—8 carbon atoms.

2. Compounds with the formula (A) as defined in Claim 1, in which X′ represents a trifluoromethyl radical.

3. Compounds with the formula (A) as defined in Claim 1 or 2 where X′ is in position 8.

4. Preparation process for the compounds with the formula (A), characterized in that a compound with the formula (II)

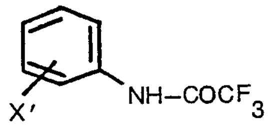

(II)

in which X′ retains the same significance as that defined in Claim 1, is submitted to the action of a chlorination agent, thus obtaining a compound with the formula (III):

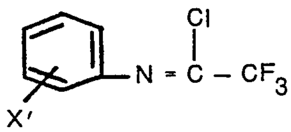

(III)

which is made to react with a derivative with the formula:

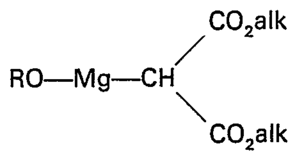

in which R and alk, identical or different, represent alkyl radicals containing 1—8 carbon atoms, or with an alkyl malonate with the formula:

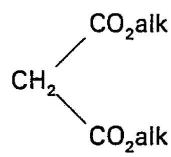

in which alk represents an alkyl radical containing 1—8 carbon atoms, in the presence of a strong base, so as to obtain a compound with the formula (IV):

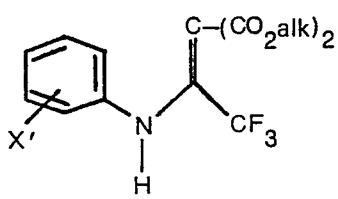

(IV)

which is cyclized so as to obtain a compound with the formula (A) in which R represents an alkyl radical containing 1—8 carbon atoms which, if necessary, is saponified so as to obtain a compound with the formula (A) in which R represents a hydrogen atom.

**Patentansprüche**

1. Verbindung der Formel (A)

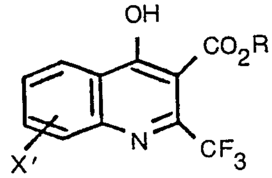

(A)

worin X′ in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, einen Trifluoromethylrest, einen Trifluoromethylthiorest oder einen Trifluoromethoxyrest bedeutet, und R ein Wasserstoffatom oder einen Alkylrest mit 1 bis 8 Kohlenstoffatomen darstellt.

2. Verbindungen der Formel (A) gemäß Anspruch 1, worin X′ einen Trifluoromethylrest bedeutet.

3. Verbindungen der Formel (A) gemäß Anspruch 1 oder 2, worin X′ sich in 8-Stellung befindet.

4. Verfahren zur Herstellung der Verbindungen der Formel (A), dadurch gekennzeichnet, daß man eine Verbindung der Formel (II)

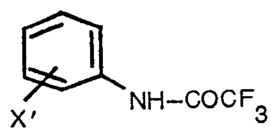

(II)

worin X′ die in Anspruch 1 angegebene Bedeutung besitzt, der Einwirkung eines Chlorierungsmittels unterzieht, so eine Verbindung der Formel (III)

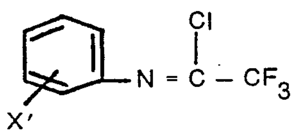

(III)

erhält, die man mit einem Derivat der Formel

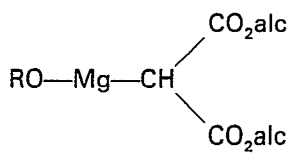

worin R und alc, die gleich oder verschieden sein können, einen Alkylrest mit 1 bis 8 Kohlenstoffatomen bedeuten oder mit einem Alkylmalonat der Formel

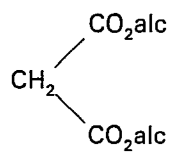

worin alc einen Alkylrest mit 1 bis 8 Kohlenstoffatomen darstellt, in Gegenwart einer starken Base umsetzt, um eine Verbindung der Formel (IV)

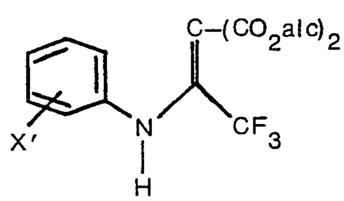

(IV)

zu erhalten, die man cyclisiert, um eine Verbindung der Formel (A) zu erhalten, worin R einen Alkylrest mit 1 bis 8 Kohlenstoffatomen bedeutet, die man gegebenenfalls verseift, um eine Verbindung der Formel (A) zu erhalten, worin R ein Wasserstoffatom bedeutet.